# EUROPÄISCHE PATENTSCHRIFT

(11) **EP 0 920 332 B1**
(45) Veröffentlichungstag und Bekanntmachung des Hinweises auf die Patenterteilung: **02.11.2005**
(21) Anmeldenummer: 98934986.5
(22) Anmeldetag: 19.06.1998
(51) Int. Cl.: A61K 38/48, A61K 31/70, A61P 13/12

(54) **KOMBINATION DER PROTEOLYTISCHEN ENZYME TRYPSIN, BROMELAIN UND PAPAIN ZUR BEHANDLUNG VON GLOMERULONEPHRITIS**
COMBINATION OF THE PROTEOLYTIC ENZYMES TRYPSIN, BROMELAIN AND PAPAIN FOR TREATING GLOMERULONEPHRITIS
COMBINAISON DES ENZYMES PROTEOLYTIQUES TRYPSIN, BROMELAIN ET PAPAIN POUR TRAITER LA GLOMERULONEPHRITE

(30) Priorität: 20.06.1997 DE 19726253
(43) Veröffentlichungstag der Anmeldung: 09.06.1999
(73) Patentinhaber: MUCOS Pharma GmbH & Co., D-82538 Geretsried (DE)
(72) Erfinder: STAUDER, Gerhard, D-82538 Geretsried (DE); RANSBERGER, Karl, D-82402 Seeshaupt (DE)
(74) Vertreter: Grünecker, Kinkeldey, Stockmair & Schwanhäusser Anwaltssozietät
(86) Internationale Anmeldenummer: PCT/EP1998/003769
(87) Internationale Veröffentlichungsnummer: WO 1998/058667

(56) Entgegenhaltungen:
- EP-A- 0 309 602
- EP-A- 0 421 023
- WO-A-97/05244
- SEBEKOVA ET AL.: "Amelioration of the progressive course of chronic renal failure in subtotally nephrectomized rats by intraperitoneal enzyme therapy" NIEREN AND HOCHDRUCKKRANKHEITEN, Bd. 26, Nr. 6, Juni 1997, Seiten 277-281, XP002082401
- NAKAZAWA ET AL.: "Proteolytic enzyme treatment reduces glomerular immune deposits and proteinuria in passive Heymann nephritis" JOURNAL EXPERIMENTAL MEDICINE, Bd. 164, Nr. 6, Dezember 1986, Seiten 1973-1987, XP002082402
- EMANCIPATOR ET AL.: "Oral enzymes in different animal models of glomerulonephritis" INTERNATIONAL JOURNAL OF IMMUNOTHERAPY, Bd. 13, Nr. 3-4, 1997, Seiten 97-103, XP002082403
- SEBEKOVA ET AL.: "Systemic treatment with proteolytic enzymes in rat models of nonimmune mediated renal diseases" INTERNATIONAL JOURNAL OF IMMUNOTHERAPY, Bd. 13, Nr. 3-4, 1997, Seiten 79-83, XP002082404
- SEBEKOVA ET AL.: "Effects of protease therapy in the remnant kidney model of progressive renal failure" MINERAL AND ELECTROLYTE METABOLISM, Bd. 23, Nr. 3-6, Mai 1997 - Dezember 1997, Seiten 291-295, XP002082405

## Beschreibung

Die vorliegende Erfindung betrifft die Verwendung von einer Kombination der proteolytischen Enzyme Trypsin, Biomelain und Papain und gegebenenfalls Rutin zur Herstellung eines Medikamentes zur Behandlung von Glomerulonephritis.

Glomerulonephritis ist ein Sammelbegriff für sehr verschiedenartige, bakterielle, Nierenerkrankungen mit Entzündungsvorgängen in den Nierenkörperchen und sekundär in anderen Teilen der Nephrone und des Interstitiums. Während einer Glomerulonephritis kann es zur Einlagerung von Imrnunkomplexablagerungen innerhalb der Gewebe kommen. Experimentelle Modelle der membranösen Glomerulonephritis können eine schwere Proteinurie, oft begleitet von einem nephrotischen Syndrom, auslösen. Häufig kommt es bei der Serum-Nephritis und der autologen Phase der Heymann Nephritis auch zu einer Hypercholesterolämie. Eine Form der aktiven Serum-Nephritis, die durch Immunisierung von Mäusen mit Dextranen induziert wird, löst eine spontane IgA-Nephropathie bei Patienten aus. Daraufhin akkumulieren sich überzählige, mesangiale Ablagerungen und einige kapilläre Ablagerungen, hauptsächlich mit IgA, in den Glomeruli. Es tritt außerdem eine signifikante Hämaturie und manchmal eine Proteinurie auf.

Da die Glomerulonephritis also mit einer Vielzahl emster Probleme für den Körper einhergeht und eine Therapie in einigen Fällen schwierig ist, werden Anstrengungen in vielen Richtungen unternommen, um die Krankheit zu heilen oder doch zumindest ihre Symptome und Beschwerden zu verringern.

Neben einer Antibiotikatherapie wird daher ein verstärktes Augenmerk auf unterstützende Mittel gerichtet, die die Behandlung ergänzen oder verstärken.

Die Behandlung verschiedener Krankheitszustände mit Proteinasen ist seit einiger Zeit bekannt. Solche Proteinasen sind z.B. Papain, Trypsin und Bromelain.

Ohne an eine Theorie gebunden zu sein, wird angenommen, daß die Wirkung von Proteinasen in der Regel auf einem Eingriff in das Immunsystem beruht.

Der vorliegenden Erfindung lag das technische Problem zugrunde, ein weiteres Medikament zur Behandlung von Glomerulonephritis zur Verfügung zu stellen. Dieses Problem wird erfindungsgemäß gelöst durch die in Anspruch 1 angegebene Erfindung. Die Unteransprüche betreffen bevorzugte Ausführungsformen der Erfindung.

Überraschend wurde gefunden, daß Proteinasen, gegebenenfalls in Kombination mit Rutin auch bei einer Krankheit wie der Glomerulonephritis wirken.

Gemäß Anspruch 1 der vorliegenden Erfindung ist die Verwendung einer Kombination der proteolytischen Enyzme Trypsin, Bromelain und Papain und gegebenenfalls Rutin zur Herstellung eines Medikaments zur Behandlung von Glomerulonephritis vorgesehen.

Es wird angenommen, daß die enzymatische Auflösung der glomerulären Immunablagerungen dem therapeutischen Effekt zugrunde liegen, obwohl andere Mechanismen, wie z.B. eine veränderte zelluläre Expression von Cytokinen und Cytokinrezeptoren, von endogenen Gewebemetalloproteasen und zellulären Adhäsionsmolekülen ebenfalls potentielle Ziele der Proteasen sind, wie auch eine Reduktion der hyperaktiven T-Zellen.

Die erfindungsgemäß verwendeten Enzyme lassen sich kostengünstig aus den folgenden Rohmaterialien isolieren.

Bromelain ist ein proteolytisches wirksames Enzym aus dem Preßsaft der Ananas und kann auch noch aus reifen Früchten isoliert werden.

Papain ist ein proteolytisches Enzym, das aus dem Milchsaft der unreifen, fleischigen Früchte des Melonenbaums Carica Papaya gewonnen wird. Reines Papain ist ein kristallines Polypeptid mit einem MG. von 23350, das aus einer Kette von 212 Aminosäureresten mit 4 Disulfid-Brücken besteht; die Sequenz und Raumstruktur sind bekannt. Papain wird vielfältig eingesetzt: Aufgrund seiner Protein-spaltenden Eigenschaft als "Fleischzartmacher" oder "Mürbesalz", zum Klären von Bier, zur Brot- und Hartkeksherstellung, in der Lederzubereitung, in der Textil-Industrie zum Entbasten von Seide und zur Verhinderung von Wollverfilzung, in der Tabak-Industrie zur Qualitätsverbesserung, zur Rückgewinnung von Silber aus verbrauchtem photographischem Material, femer in der Bakteriologie zur Pepton-Gewinnung. In der Medizin dient Papain bereits zur Unterstützung der enzymatischen Verdauung, zur enzymatischen Wundreinigung und als Zusatz zu Zahnprothese-Reiniungsmitteln. Für Spezialzwecke werden Papain-Präparate auch an Kunststoffpolymere oder an Agaroseträger gebunden angeboten. Papain ist auch als Katalysator zur Synthese von Oligopeptiden verwendet worden.

Trypsin ist ein proteolytisches Enzym, das ebenfalls im Pankreas gebildet wird und in Verbindung mit anderen Enzymen bereits therapeutisch eingesetzt wird. Es gehört zu den Serin-Proteinasen. Kristallines Trypsin hat ein MG. von ca. 23300, ist in Wasser, nicht aber in Alkohol löslich, besitzt ein Wirkungsoptimum bei pH 7-9 und spaltet Peptid-Ketten spezifisch Carboxy-seitig der basischen Aminosäurereste L-Lysin und L-Arginin. Die räumliche Struktur des aus 223 Aminosäuren bestehenden Trypsins ist bekannt.

Weiterhin kann zusätzlich Rutosid dem Medikament beigemischt werden. Rutosid, auch als Rutin bekannt, ist ein Glycosid, das zu den Flavonoiden gehört.

Eine besonders gute Wirksamkeit zeigt sich bei der Verwendung einer Kombination der Enzyme Bromelain, Papain und/oder Trypsin. Neben der bemerkenswerten und unerwarteten Wirkung dieser Enzyme auf die Verbesserung eines Glomerulonephritis-Krankheitszustan-des hat die kombinierte Verwendung der genannten Enzyme weiterhin den Vorteil, daß auch bei einer Langzeitanwendung keine schädigenden Nebenwirkungen auftreten.

Eine besonders gute Wirksamkeit hat die kombinierte Verwendung von 20 bis 100 mg Bromelain, 40 bis 120 mg Papain und 10 bis 50 mg Trypsin pro Dosiseinheit.

Bevorzugte Ausführungsformen werden in den Ansprüche 2 und 3 beschrieben.

In einer weiteren bevorzugten Ausführungsform werden 10 bis 100 mg, besonders bevorzugt 100 mg Rutosid x 3 H₂O pro Dosiseinheit verwendet.

Das Medikament kann weiterhin alle üblichen Hilfs- und/oder Trägerstoffe enthalten.

Als Hilfs- und Trägerstoffe kommen z.B. Lactose, Magnesiumstearat, Stearinsäure, Talkum, Methacrylsäure, Copolymerisat Typ A, Shellack, Makrogol 6000, Dibutylphthalat, Vanillin, Titandioxid, weißer Ton, Polyindon, gelbes Wachs und Camaubawachs in Frage.

Die folgenden Beispiele sollen die Erfindung im einzelnen erläutern.

### Beispiel 1

### Material und Methoden

### 1. Material

Es wurde das Zellkulturmedium RPMI 1640 mit folgenden Zusätzen verwendet: NaHCO₃ (Biochrom, 2 g/1); L-Glutamin (Biochrom, 2 mM), Na-Pyruvat (Biochrom, 1 mM), NaN₃ (Sigma, 0,01 %).

Im Falle einer Stimulation der Zellen wurde entweder Phythämagglutinin M (Biochrom, 5 µg/ml) oder γ-Interferon (100 U/ml) eingesetzt. Die Stimulation der Zellen erfolgte dabei über 1-3 Tage mit Zusatz von 10 % fötalem Kälberserum (Biochrom).

Als Targets für die zu untersuchenden Enzyme wurden frisch isolierte, humane, periphere, mononukleäre Blutzellen (Citratblut) verwendet. Nach der üblichen Isolation mittels Ficoll wurden die Zellen mehrfach gewaschen und frisch bei den Experimenten eingesetzt. Neutrophile Granulozyten wurden ebenfalls aus frischem Citratblut isoliert. Hierbei erfolgte die Abtrennung von den LymphozytenlMonozyten mittels eines 2-Stufen-Ficoll-Gradienten.

### 2. Verwendete monoklonale Antikörper

Für die spezifische Erkennung der Oberflächenstrukturen der Leukozyten wurden monoklonale Antikörper verwendet. Diese erkennen auf den entsprechenden Antigenen jeweils ein definiertes Epitop, welches bei den von uns untersuchten Antigenen nur ein einziges Mal in der Struktur vorkommt. In Übersicht 1 sind die untersuchten Oberflächenmarker, die monoklonalen Antikörper sowie die analysierten Targetzellen dargestellt.

**Übersicht 1:**

| Analysierte Oberflächenmarker, verwendete monoklonale Antikörper und Targetzellen der Enzymbehandlung | | | | |
|---|---|---|---|---|
| Marker | Epitop AK-Bez. | Fluorochrom | Produzent | Target-Zelle |
| CD2 | Leu5b | PE¹ | B.D.³ | T-Lymphozyten |
| CD4 | Leu3a | PE | B.D. | T-Lymphozyten |
| | OKT4 | FITC² | Ortho⁴ | T-Lymphozyten |
| CD11b | Leu15 | PE | B.D. | Granulozyten |
| CD25 | IL-2R | PE | B.D. | PHA-Blasten |

| | | | | |
|---|---|---|---|---|
| ¹ Phycoerythrin, rote Fluoreszenz; | | | | |
| ² Fluoreszeinisothiocyanat, grüne Fluoreszenz; | | | | |
| ³ Becton Dickinson, Heidelberg; | | | | |
| ⁴ Ortho Diagnostics | | | | |

### 3. Inkubationsbedingungen

Die frisch isolierten und aufbereiteten Zellen wurden mit den Enzymen Bromelain, Papain und Trypsin (Arzneimittel-Inhaltsstoffe der Fa. Mucos) mit den jeweils in den Legenden der Tabellen und Abbildungen angegebenen Konzentrationen inkubiert. Bei der Mischung der drei Enzyme entsprach das Mischungsverhältnis 22,7 : 15,5: 11,9 (Bromeiain : Papain : Trypsin, bezogen auf 40 µg/ml, "BPT"). Es wurden drei Enzymkonzentrationen (40, 10, 2,5 µg/ml) untersucht. Die Inkubation fand in serumfreiem Medium bei 37°C statt.

Die Proteasen wurden unmittelbar vor den Inkubationen angesetzt. In den Zellkulturmedien war 0,01 % Natriumazid enthalten. Durch diesen Zusatz werden die Zellen daran gehindert, während des Inkubationsprozesses oder der Waschvorgänge Rezeptormoleküle erneut zu exprimieren. Nach dem Auswaschen des Zellkulturmediums sind die Zellen wieder aktivierbar (nicht demonstriert).

Nach entsprechender Inkubation der Zellen mit den jeweiligen Enzymen, Auswaschen der Enzyme und der Markierung mit monoklonalen Antikörpern (nach Angaben der Hersteller) wurde sofort die Analyse der Oberflächenmarker vorgenommen.

### 4. Analytische Durchflußcytometrie

Alle Untersuchungen zur Modulation von Zelloberflächenmolekülen wurden mittels analytischer Durchflußcytometrie (FACSCAN, Fa. Becton Dickinson, Heidelberg) unter Verwendung einer gerätespezifischen Software (Lysis I) durchgeführt. Bei entsprechender Einstellung des Gerätes sowie der Mitführung von Referenzen, d.h. Zellen, die ohne Enzyme aber in derselben Prozedur behandelt wurden, erfolgte die Messung.

Für jede Subklasse der monoklonalen Antikörper wurde eine entsprechende fluoreszenzkonjugierte Isotypenkontrolle mitgeführt. Hierbei handelt es sich um Maus-Immunglobulin, mit welchem die Kapazität der unspezifischen Bindung der Targetzellen flußcytometrisch erfaßt wird.

Pro Histogramm wurden 10 000 Zellen gemessen. Die jeweilige Zellpopulation wurde mit einem sogenannten "elektronischen Gate" separiert, in dem sich dann mindestens 3 500 Zellen befanden.

### 5. Darstellung der Ergebnisse

Die Auswertung und Analyse der Daten erfolgte unabhängig vom Meßvorgang auf dem Durchflußcytometer mittels einer gerätespezifischen Software. Dabei wurden jeweils die Histogramme der Kontrollen, d. h. der unbehandelten Zellproben, mit den Histogrammen der enzymbehandelten Zellen verglichen.

Die Rohdarstellung umfaßt ein optisch eindrucksvolles, aber relativ unübersichtliches Histogramm, bei dem verschiedene Einzelmessungen übereinander gelagert abgebildet sind. Hier läßt sich insbesondere die Wirkung der Enzyme im Vergleich zur Referenz optisch transparent machen. Für diese Untersuchungen ist nicht der prozentuale Anteil einer Subpopulation an der gesamten Leukozytenpopulation die Meßgröße, sondern die relative Rezeptordichte, die sich als Fluoreszenzintensität darstellt.

Aus diesen Histogrammen, die beispielhaft sind und illustrativen Charakter haben, lassen sich Daten ableiten, welche die relative Fluoreszenzintensität der Einzelmessung reflektieren. Diese ist ein Maß für die relative Rezeptor- oder Oberflächenmoleküldichte bei einer gemessenen Zellpopulation.

Die Säulengraphiken zeigen in logarithmischer Darstellung den Median der relativen Fluoreszenz. Es läßt sich so gegenüber der Referenz die Reduktion der Dichte des jeweiligen Oberflächenmoteküls in Abhängigkeit von der Enzymkonzentration darstellen.

In den Tabellen sind die Daten unabhängiger Experimente enthalten. Die Nummern der Spender haben nur für die jeweilige Tabelle Gültigkeit und sind nicht auf andere Tabellen übertragbar. Wird in der Tabelle beispielsweise ein Wert von 40 % angegeben, so bedeutet das, daß bei diesem Antigen 40 % aller Oberflächenmoleküle durch das Enzym so verändert ist, daß der spezifische monoklonale Antikörper sein Epitop nicht mehr erkennt. Wird keine Reduktion beobachtet, so erscheint in den Tabellen der Wert "0". Die angegebene Prozentzahl drückt somit die Enzymleistung gegenüber den einzelnen Antigenen aus. Werte bis zu 20 % werden im Einzelfall als nicht relevant angesehen.

Für eine Bewertung der Wirkung der Enzyme auf die einzelnen Antigene ist es günstig, einen geeigneten Vergleichsmaßstab zu wählen. Bis auf wenige Ausnahmen wurden alle Untersuchungen unter standardisierten Inkubationsbedingungen durchgeführt. Damit kann für diese experimentellen Bedingungen die aus früheren Forschungsberichten bekannte Halbeffektkonzentration angegeben werden.

Zur Berechnung der Halbeffektkonzentration wurden die Daten mittels nichtlinearer Regression ausgewertet. Der Median der Fluoreszenzintensität versus eingesetzter Enzymkonzentration und Referenz werden zueinander in Beziehung gesetzt, und daraus läßt sich die Menge an Enzym berechnen, die zu einer 50%igen Reduktion der relativen Fluoreszenzintensität bzw. der in der Struktur veränderten Rezeptordichte führt.

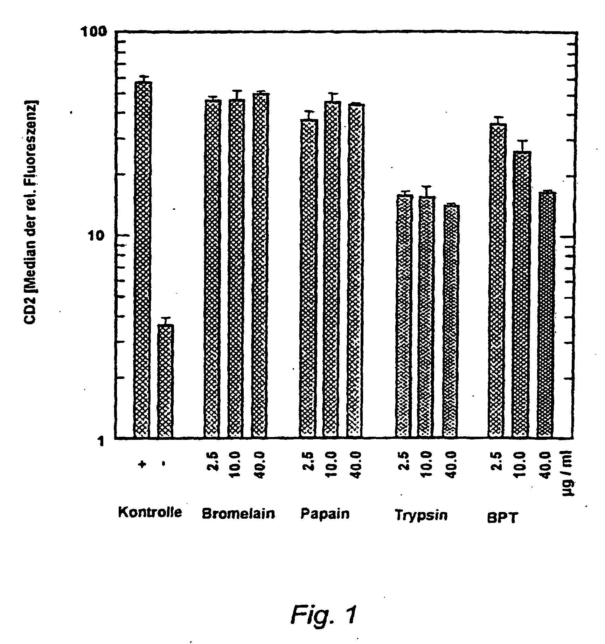

**Abbildung 1:** CD2-Modulation durch Proteasen; Angegeben ist der Median der relativen Fluoreszenzintensität; Targetzellen: Lymphozyten. Die Positivkontrolle (Referenz) sind unbehandelte Zellen, die mit dem für CD2 spezifischen monoklonalen Antikörper inkubiert wurden. Die Negativkontrolle sind unbehandelte Zellen, die mit dem Antikörper-Isotyp inkubiert wurden. Die Inkubationszeit mit den Enzymen betrug 60 min. Dargestellt sind die Daten von Spender 1 (vgl. Tab. 1) als Mittelwert von Doppelbestimmungen und Standardabweichung.

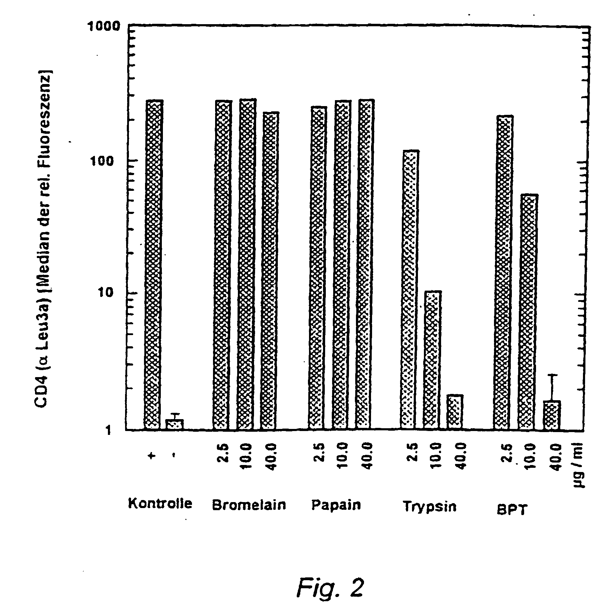

**Abbildung 2:** CD4 (Epitop Leu3a)-Modulation durch Proteasen; Angegeben ist der Median der relativen Fluoreszenzintensität; Targetzellen: Lymphozyten. Die Positivkontrolle (Referenz) sind unbehandelte Zellen, die mit dem für CD4 spezifischen, monoklonalen Antikörper inkubiert wurden. Die Negativkontrolle sind unbehandelte Zellen, die mit dem Antikörper-Isotyp inkubiert wurden. Die Inkubationszeit mit den Enzymen betrug 60 min. Dargestellt sind die Daten von Spender 2 (vgl. Tab. 2) als Mittelwert von Doppelbestimmungen und Standardabweichung.

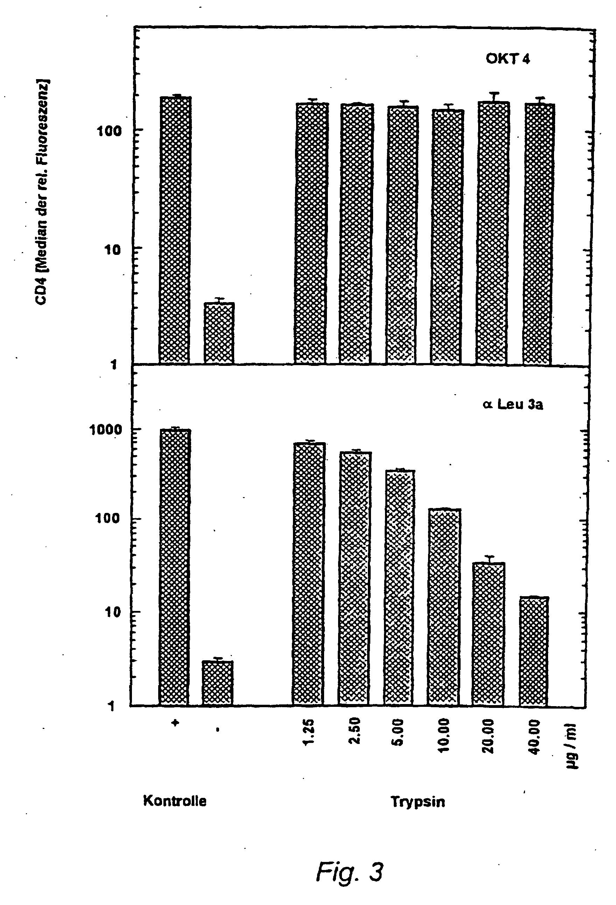

**Abbildung 3:** CD4 (Epitope OKT4 und Leu3a)-Modulation durch Trypsin; Angegeben ist der Median der relativen Fluoreszenzintensität; Targetzellen: Lymphozyten. Die Positivkontrolle (Referenz) sind unbehandelte Zellen, die mit dem für CD4 spezifischen monoklonalen Antikörper inkubiert wurden. Die Negativkontrolle sind unbehandelte Zellen, die mit dem Antikörper-Isotyp inkubiert wurden. Die Inkubationszeit mit den Enzymen betrug 60 min. Dargestellt sind die Daten von einem Spender (vgl. Tab. 3) als Mittelwert von Doppelbestimmungen und Standardabweichung.

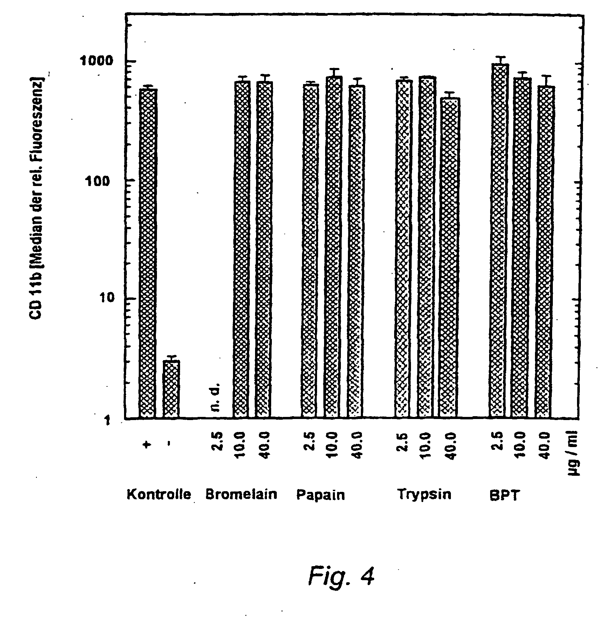

**Abbildung 4:** CD11b-Modulation durch Proteasen; Angegeben ist der Median der relativen Fluoreszenzintensität; Targetzellen: Granulozyten. Die Positivkontrolle (Referenz) sind unbehandelte Zellen, die mit dem für CD11 b spezifischen monoklonalen Antikörper inkubiert wurden. Die Negativkontrolle sind unbehandelte Zellen, die mit dem Antikörper-Isotyp inkubiert wurden. Die Inkubationszeit mit den Enzymen betrug 60 min. Dargestellt sind die Daten von einem Spender als Mittelwert von Doppelbestimmungen und Standardabweichung.

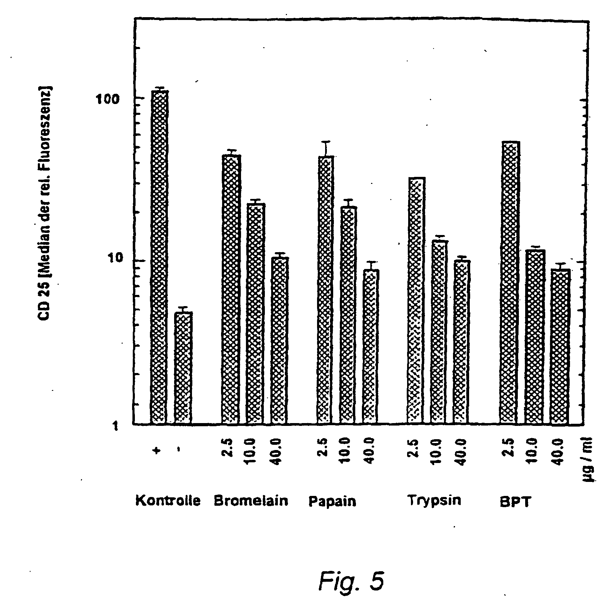

**Abbildung 5:** CD25-Modulation durch Proteasen; Angegeben ist der Median der relativen Fluoreszenzintensität; Targetzellen: PHA-Blasten. Die Positivkontrolle (Referenz) sind unbehandelte Zellen, die mit dem für CD25 spezifischen, monoklonalen Antikörper inkubiert wurden. Die Negativkontrolle sind unbehandelte Zellen, die mit dem Antikörper-Isotyp inkubiert wurden. Die Inkubationszeit mit den Enzymen betrug 60 min. Dargestellt sind die Daten von Spender 1 als Mittelwert von Doppelbestimmungen und Standardabweichung.

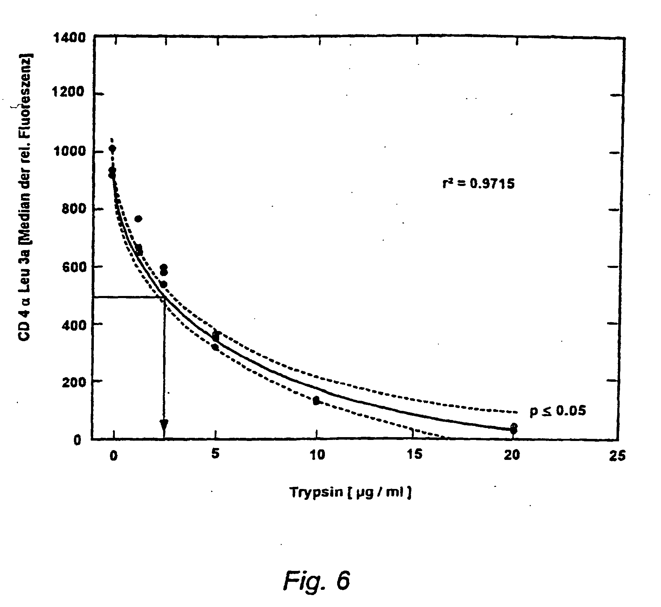

**Abbildung 6:** Reduktion der Leu3a-Antigendichte durch Trypsin. Frisch isolierte humane periphere, mononukleäre Blutzellen wurden mit Trypsin im serumfreien Medium inkubiert, anschließend gewaschen und mit dem monoklonalen Antikörper anti-Leu3a markiert. Die Reduktion der relativen Fluoreszenzdichte von CD4 der Lymphozytenpopulation ist das Maß der Enzymaktivität. Die Halbeffektkonzentration von Trypsin gegenüber dem Epitop Leu3a von CD4 wird über die gefittete Kurve berechnet.

### Ergebnisse

**Tabelle 1:**

| CD2-Modulation durch Proteasen; Targetzellen: Lymphozyten. Angegeben ist die prozentuale Reduktion des Mediums der relativen Fluoreszenzintensität, die ein Maß der Enzymteistung ist. Die Inkubationszeit mit den Enzymen betrug 60 min. Es sind die Ergebnisse von drei Experimenten mit Zellen von drei verschiedenen Spendern dargestellt. | | | | |
|---|---|---|---|---|
| Enzym | Nr. | 40 µg/ml | 10 µg/ml | 2,5 µg/ml |
| Bromelain | 1 | 11,1 | 17,9 | 17,4 |
| | 2 | 6,1 | 12,2 | 14,3 |
| | 3 | 0 | 0 | 0 |
| Papain | 1 | 22,4 | 21,4 | 34,7 |
| | 2 | 5,4 | 9,5 | 17,0 |
| | 3 | 0 | 0 | 0 |
| Trypsin | 1 | 75,7 | 73,6 | 73,0 |
| | 2 | 83,7 | 21,1 | 0,7 |
| | 3 | 96,3 | 85,4 | 50,9 |
| BPT | 1 | 71,9 | 54,7 | 38,2 |
| | 2 | 74,1 | 8,8 | 18,4 |
| | 3 | 94,8 | 72,6 | 25,2 |

**Tabelle 2:**

| CD4(Epitop Leu3a)-Modulation durch Proteasen; Targetzellen: Lymphozyten. Angegeben ist die prozentuale Reduktion des Medians der relativen Fluoreszenzintensität, die ein Maß der Enzymleistung ist. Die Inkubationszeit mit den Enzymen betrug 60 min. Es sind die Ergebnisse von zwei Experimenten mit Zellen von zwei verschiedenen Spendern dargestellt. | | | | |
|---|---|---|---|---|
| Enzym | Nr. | 40 µg/ml, | 10 µg/ml | 2,5 µg/ml |
| Bromelain | 1 | 24,6 | 0 | 0 |
| | 2 | 16,2 | 0 | 0 |
| - Papain | 1 | 2,5 | 3,2 | 0 |
| | 2 | 0 | 0 | 5,7 |
| Trypsin | 1 | 99,3 | 93,0 | 64,1 |
| | 2 | 99,4 | 96,2 | 57,5 |
| BPT | 1 | 98,3 | 49,3 | 23,0 |
| | 2 | 99,4 | 79,2 | 20,2 |

**Tabelle 3:**

| Modulation der CD4-Epitope Leu3a und OKT4 durch Trypsin; Targetzelten: Lymphozyten. Angegeben ist die prozentuale Reduktion des Medians der relativen Fluoreszenzintensität, die ein Maß der Enzymleistung ist. Die Inkubationszeit mit den Enzymen betrug 60 min. Es sind die Daten von einem Spender dargestellt. | | | | |
|---|---|---|---|---|
| Enzym | Epitop | 40 µg/ml | 20 µg/ml | 10 µg/ml |
| Trypsin | Leu3a | 98,5 | 96,7 | 87,1 |
| | OKT4 | 6,5 | 6,3 | 19,5 |
| | Epitop | 5 µg/ml | 2,5 µg/ml | 1,25 µg/ml |
| Trypsin | Leu3a | 65,2 | 41,9 | 28,8 |
| | OKT4 | 13,3 | 10,8 | 9,3 |

**Tabelle 4:**

| CD11b-Modulation durch Proteasen; Targetzellen: Granulozyten. Angegeben ist die prozentuale Reduktion des Medians der relativen Fluoreszenzintensität, die ein Maß der Enzymleistung ist. Die Inkubationszeit mit den Enzymen betrug 60 min. Es sind die Ergebnisse von drei Experimenten mit Zellen von drei verschiedenen Spendern dargestellt. | | | | |
|---|---|---|---|---|
| Enzym | Nr. | 40 µg/ml | 10 µg/ml | 2,5 µg/ml |
| Bromelain | 1 | 0 | 0 | 26,4 |
| | 2 | 0 | 0 | n.d. |
| | 3 | 13,5 | 8,1 | 27,5 |
| Papain | 1 | 0 | 0 | 0 |
| | 2 | 0 | 0 | 0 |
| | 3 | 7,9 | 35,9 | 20,0 |
| Trypsin | 1 | 0 | 0 | n.d. |
| | 2 | 17, 0 | 0 | 0 |
| | 3 | 19,0 | 1,9 | 13,9 |
| BPT | 1 | 0 | 4,3 | n.d. |
| | 2 | 0 | 0 | 0 |
| | 3 | 3,1 | 8,6 | 4,7 |

**Tabelle 5:**

| CD25-Modulation durch Proteasen; Targetzellen: Lymphozyten, Monozyten, NK-Zellen. Angegeben ist die prozentuale Reduktion des Medians der relativen Fluoreszenzintensität, die ein Maß der Enzymleistung ist. Die Inkubationszeit mit den Enzymen betrug 60 min. Es sind die Ergebnisse von zwei Experimenten mit Zellen von zwei verschiedenen Spendern dargestellt. Vor dem Experiment wurden die Zellen 3 Tage mitogenstimuliert. | | | | |
|---|---|---|---|---|
| Enzym | Nr. | 40 µg/ml | 10 µg/ml | 2,5 µg/ml |
| Bromelain | 1 | 90,7 | 80,1 | 59,7 |
| | 2 | 62,6 | 43,6 | 0 |
| Papain | 1 | 92,2 | 81,0 | 60,7 |
| | 2 | 62,6 | 43,6 | 0 |
| Trypsin. | 1 | 91,2 | 88,2 | 71,0 |
| | 2 | 78,9 | 73,9 | 52,8 |
| BPT | 1 | 92,1 | 89,7 | 50,9 |
| | 2 | 79,4 | 44,1 | 12,2 |

**Tabelle 6:**

| Berechnete Halbeffektkonzentration (µg/ml) von Bromelain, Papain, Trypsin und deren Kombination für die 50 %ige Reduktion der Dichte von zellulären Oberflächenmolekülen. Die Enzyminkubation betrug 1 Stunde. | | | |
|---|---|---|---|
| Enzyme | CD2 | CD4³ | CD25⁴ |
| Bromelain Halbeffektkonz.¹ | n w | n w | 18,4 |
| Bereich² | - | - | 14-23 |
| Papain Halbeffektkonz.¹ | n w | n w | 12,4 |
| Bereich² | - | - | 11-14 |
| Trypsin Halbeffektkonz.¹ | 1,5 | 2,5 | < 2,5 |
| Bereich² | 1-2 | 2,3-3,1 | |
| B-P-T⁵ Halbeffektkonz.¹ | 9,3 | 16,4 | 16,0 |
| Bereich² | 7,5-14 | 15,5-18 | 13-19 |

| | | | |
|---|---|---|---|
| ¹ berechnet aus Mittelwerten von 2 oder 3 unabhängigen Experimenten | | | |
| ² minimaler und maximaler Wert, 95 % Konfidenzbereich = 1 δ, geschätzt | | | |
| ³ Modulation von CD4-Epitop Leu3a | | | |
| ⁴ auf stimulierten Zellen präsent | | | |
| ⁵ Mischung der Proteasen im Verhältnis 22,7 : 15,5 : 11,9 - Bromeiain : Papain : Trypsin n w: nicht wirksam im untersuchten System (max 40 µg Enzym/ml, 1 h Inkubation) | | | |

## Patentansprüche

1. Verwendung einer Kombination der proteolytischen Enzyme Trypsin, Bromelain und Papain zur Herstellung eines Medikamentes zur Behandlung von Glomeruionephritis, **dadurch gekennzeichnet, dass** 20 bis 100 mg Bromelain, 40 bis 120 mg Papain und 10 bis 50 mg Trypsin pro Dosiseinheit verwendet werden, und dass gegebenenfalls zusätzlich Rutosid verwendet wird

2. Verwendung nach Anspruch 1, **dadurch gekennzeichnet, dass** 90 mg Bromelain, 120 mg Papain und 100 mg Rutosid pro Dosiseinheit verwendet werden.

3. Verwendung nach Anspruch 1, **dadurch gekennzeichnet, dass** 90 mg Bromelain, 48 mg Trypsin und 100 mg Rutosid pro Dosiseinheit verwendet werden.

4. Verwendung nach Anspruch 1, **dadurch gekennzeichnet, dass** 10 bis 100 mg, vorzugsweise 100 mg Rutosid x 3 H₂O pro Dosiseinheit verwendet werden.

## Claims

1. Use of a combination of the proteolytic enzymes trypsin, bromelain and papain for producing a medicament for the treatment of glomerulonephritis, **characterized in that** 20 to 100 mg of bromelain, 40 to 120 mg of papain and 10 to 50 mg of trypsin are used per dose unit, and **in that** where appropriate rutoside is additionally used.

2. Use according to Claim 1, **characterized in that** 90 mg of bromelain, 120 mg of papain and 100 mg of rutoside are used per dose unit.

3. Use according to Claim 1, **characterized in that** 90 mg of bromelain, 48 mg of trypsin and 100 mg of rutoside are used per dose unit.

4. Use according to Claim 1, **characterized in that** 10 to 100 mg, preferably 100 mg of rutoside x 3 H₂O are used per dose unit.

## Revendications

1. Utilisation d'une combinaison d'enzymes protéolytiques trypsine, bromélaïne et papaïne, pour la préparation d'un médicament afin de traiter la glomérulonéphrite, **caractérisée en ce que** l'on utilise 20 à 100 mg de bromélaïne, 40 à 120 mg de papaïne et 10 à 50 mg de trypsine par unité de dosage et on emploie éventuellement aussi du rutoside.

2. Utilisation selon la revendication 1, **caractérisée en ce que** l'on utilise 90 mg de bromélaïne, 120 mg de papaïne et 100 mg de rutoside par unité de dosage.

3. Utilisation selon la revendication 1, **caractérisée en ce que** l'on utilise 90 mg de bromélaïne, 48 mg de trypsine et 100 mg de rutoside par unité de dosage.

4. Utilisation selon la revendication 1, **caractérisée en ce que** l'on utilise 10 à 100 mg, de préférence 100 mg de rutoside x 3 H₂O par unité de dosage.
